Europäisches Patentamt

European Patent Office

Office européen des brevets

(11) Publication number: **0 115 114**
**B1**

(12) EUROPEAN PATENT SPECIFICATION

(45) Date of publication of patent specification: 16.07.86

(51) Int. Cl.⁴: **C 07 D 231/40**

(21) Application number: 83304851.5

(22) Date of filing: 23.08.83

(54) Polymorphic forms of 5-(3-(2-(2,2,2-trifluoroethyl)-guanidino)-pyrazol-1-yl) valeramide.

(30) Priority: 03.09.82 GB 8225154

(43) Date of publication of application:
08.08.84 Bulletin 84/32

(45) Publication of the grant of the patent:
16.07.86 Bulletin 86/29

(84) Designated Contracting States:
AT BE CH DE FR GB IT LI LU NL SE

(56) References cited:
EP-A-0 060 094

(73) Proprietor: IMPERIAL CHEMICAL INDUSTRIES PLC
Imperial Chemical House Millbank
London SW1P 3JF (GB)

(72) Inventor: Hardie, Janet
7 Radnor Close
Congleton Cheshire (GB)
Inventor: Platt, Ronald
10 Worthington Close
Henbury Macclesfield Cheshire (GB)
Inventor: Hutton, Jonathan
Penrhyn Cottage Brookledge Lane
Adlington Cheshire (GB)
Inventor: Warren, Peter
8 Penrith Court
Congleton Cheshire (GB)
Inventor: Lee, Stanley Arnold
4 Manor Crescent
Macclesfield Cheshire (GB)
Inventor: Harris, Gregory David
55 Wellington Road Bollington
Macclesfield Cheshire (GB)
Inventor: Davies, Elwyn Peter
Flat 12, Rugby House Brocklehurst Way
Macclesfield Cheshire (GB)

**0 115 114**

(74) Representative: **Brown, Ivor James Stewart et al**
**Imperial Chemical Industries PLC Legal Department: Patents PO Box 6 Welwyn Garden City Herts, AL7 1HD (GB)**

## Description

This invention relates to three polymorphic forms of 5 - [3 - (2 - [2,2,2 - trifluoroethyl]guanidino) - pyrazol - 1 - yl]valeramide, two of which are practically crystallographically pure, and to processes for their manufacture.

The compound 5 - [3 - (2 - [2,2,2 - trifluoroethyl] - guanidino)pyrazol - 1 - yl]valeramide is a histamine H-2 receptor antagonist whose preparation and antagonist activity are described in European Patent Publication No. 60094. The compound is conveniently referred to by the code number ICI 162846. It has now been discovered that ICI 162846 exists in three polymorphic forms, and that two of these can be obtained in a practically pure crystallographic form by employing suitable techniques of recrystallisation. The polymorphic forms are conveniently referred to as ICI 162846 polymorph A, ICI 162846 polymorph B and ICI 162846 polymorph C. Each of forms A and B has an advantage over the others. Thus polymorph B forms larger crystals which are extremely easy to isolate by filtration. Polymorph A forms smaller crystals which are however still easy to isolate by filtration because of their ability to aggregate. In addition, polymorph A is more crystallographically stable than polymorph B or C, and as a result it is easier to obtain and identify as chemically and crystallographically pure. For these reasons polymorph A is preferred.

According to this invention there is provided ICI 162846 polymorph A having a crystallographic purity of at least 90% and which is characterised by an attenuated total reflection (ATR) infra-red spectrum having bands at 1685 (ms), 1560 (w), 1540 (m), 1455 (w), 1060 (mw), 832 (m), 788 (w) and 365 (m) cm$^{-1}$.

According to a further feature of the invention there is provided ICI 162846 polymorph B having a crystallographic purity of at least 90% and which is characterised by an attenuated total reflection (ATR) infra-red spectrum having bands at 1665 (ms), 1550 (s), 1445 (m), 1360 (w), 1215 (mw), 1070 (w), 1050 (mw), 990 (w), 935 (w), 822 (m), 380 (m) and 350 (mw) cm$^{-1}$.

The purity threshold of 90% refers to the fact that no more than 10% of the other two polymorphic forms is present. The preferred purity threshold is 95%.

It is also to be understood that the contractions s, ms, m, mw and w are well-known to infra-red spectroscopists and have the following meanings relating to the intensity of any one band:—

s—strong
ms—medium strong
m—medium
mw—medium weak
w—weak

A band which is particularly useful for characterising ICI 162846 polymorph A is that at 1685 cm$^{-1}$ and a band which is particularly useful for characterising polymorph B is that at 1665 cm$^{-1}$.

The two polymorphs may also be differentiated by means of their X-ray powder diffraction patterns, as shown in the following table.

| ICI 162846 Polymorph A | | ICI 162846 Polymorph B | |
|---|---|---|---|
| d spacing, Å | Approximate relative intensity | d spacing, Å | Approximate relative intensity |
| 12.10 | 100 | 8.58 | 11 |
| 6.34 | 11 | 8.11 | 4 |
| 5.16 | 10 | 7.72 | 6 |
| 4.98 | 12 | 6.91 | 12 |
| 4.87 | 5 | 6.39 | 4 |
| 4.50 | 21 | 6.25 | 4 |
| 4.43 | 19 | 6.08 | 5 |
| 4.24 | 43 | 6.00 | 18 |
| 4.01 | 56 | 5.22 | 54 |
| 3.84 | 5 | 5.08 | 18 |
| 3.67 | 46 | 5.02 | 16 |

TABLE (contd.)

| ICI 162846 Polymorph A | | ICI 162846 Polymorph B | |
|---|---|---|---|
| d spacing, Å | Approximate relative intensity | d spacing, Å | Approximate relative intensity |
| 3.59 | 21 | 4.55 | 24 |
| 3.54 | 16 | 4.44 | 100 |
| 3.35 | 3 | 4.32 | 10 |
| 3.22 | 3 | 4.22 | 16 |
| 2.97 | 5 | 4.13 | 30 |
| 2.91 | 5 | 4.05 | 15 |
| 2.72 | 3 | 3.92 | 6 |
| 2.64 | 2 | 3.80 | 24 |
| 2.57 | 7 | 3.73 | 21 |
| 2.52 | 2 | 3.58 | 7 |
| 2.49 | 5 | 3.44 | 8 |
| 2.34 | 3 | 3.35 | 22 |
| | | 3.29 | 8 |
| | | 3.15 | 6 |
| | | 3.12 | 16 |
| | | 3.00 | 3 |
| | | 2.86 | 6 |
| | | 2.60 | 3 |
| | | 2.56 | 3 |
| | | 2.50 | 3 |
| | | 2.38 | 2 |
| | | 2.23 | 3 |
| | | 2.19 | 3 |
| | | 2.14 | 3 |

A rapid method for establishing the presence of particular polymorphs in a sample of ICI 162846 is differential scanning calorimetry (DSC). Using this technique ICI 162846 polymorph A has a melting point of approximately 130°C., ICI 162846 polymorph B has a melting point of approximately 107°C. and ICI 162846 polymorph C has a melting point in the range 122—124°C.

According to a further feature of the invention there is provided a process for preparing ICI 162846 polymorph A having a crystallographic purity of at least 90%, and having the ATR infra-red spectrum given above, from ICI 162846 polymorph B or from a mixture of ICI 162846 polymorph A and ICI 162846 polymorph B. The process is characterised by (a) heating the starting material in a diluent at or above a temperature of 107°C; (b) recrystallising the starting material from a solution in a solvent in which crystallisation begins above 65°C., and at a cooling rate slower than 1°C./minute; (c) dissolving the starting

4

material in a boiling solution of ethyl acetate and seeding with a crystal of ICl 162846 polymorph A at about 67°C.

In process (a) the diluent must, of course, have a boiling point at or above 107°C. at ambient pressure. Suitable diluents are, for example, toluene, xylene, anisole, chlorobenzene and diethyleneglycol dimethyl ether and mixtures of any two of these. A preferred diluent is toluene.

In process (b) suitable solvents are, for example, n-butanol, isobutanol, amyl alcohol and methyl isobutyl ketone, and mixtures of any of these with toluene, xylene, anisole, chlorobenzene and diethyleneglycol dimethyl ether. The preferred solvent is toluene/n-butanol. The preferred rate of cooling the solution is 10°C./hour.

According to a further feature of the invention there is provided a process for preparing ICl 162846 polymorph B having a crystallographic purity of at least 90%, and having the ATR infra-red spectrum given above, from ICl 162846 polymorph A or from a mixture of ICl 162846 polymorph A and ICl 162846 polymorph B characterised by recrystallising the starting material from water and seeding the solution with a crystal of ICl 162846 polymorph B in the temperature range 27—37°C. It is preferable to add sufficient NaOH to the water to remove any corresponding carboxylic acid impurity.

According to a further feature of the invention there is provided a process for preparing ICl 162846 polymorph A having a crystallographic purity of at least 90%, and having the ATR infra-red spectrum given above, from a mixture containing ICl 162846 polymorph C characterised by dissolving the starting material in a solvent in which crystallisation begins above 65°C., treating this solution with an insoluble base, removing the insoluble base and allowing the solution to cool at a rate slower than 1°C./minute. The insoluble base must of course be removed before crystallisation begins. A suitable solvent for the starting material is, for example, one of those listed above for use in the preparation of polymorph A from polymorph B. The preferred solvent is toluene/n-butanol. The insoluble base may be, for example, an inorganic base such as calcium carbonate, calcium hydroxide or potassium carbonate. The preferred base is calcium hydroxide. The preferred rate of cooling of the solution is 10°C./hour.

ICl 162846 polymorph A or ICl 162846 polymorph B may be used in the form of a pharmaceutical composition which comprises polymorph A or B in association with a non-toxic pharmaceutically-acceptable diluent or carrier. Such a composition may be in a form suitable for oral, rectal, parenteral or topical administration for which purposes it may be formulated into tablets, capsules, aqueous or oily suspensions, dispersible powders, suppositories, sterile injectable aqueous or oily suspensions, gels, creams or ointments. In addition the pharmaceutical composition may also contain, or be co-administered with, one or more known drugs.

A preferred pharmaceutical composition is one suitable for oral administration, for example a tablet or capsule which contains between 2 mg. and 25 mg. of ICl 162846 polymorph A or B.

Such a pharmaceutical composition will normally be administered to man for the treatment of peptic ulcers and other conditions caused or exacerbated by gastric acidity in the same general manner as that employed for cimetidine, due allowance being made in terms of dose levels for the potency of ICl 162846 polymorph A or B relative to cimetidine. Thus each patient will receive an oral dose of between 2 and 50 mg. of ICl 162846 polymorph A or B or an intravenous, subcutaneous or intramuscular dose of between 1 mg. and 10 mg. of ICl 162846 polymorph A or B, the composition being administered 1 to 4 times, and preferably once, per day. The rectal dose will be approximately the same as the oral dose. The composition may be administered less frequently when it contains an amount of ICl 162846 polymorph A or B which is a multiple of the amount which is effective when given 1—4 times per day.

ICl 162846 may be prepared as follows:—

Sodium hydride paste (6.16 g. of 61% w/w suspension in liquid paraffin) was added portionwise over 30 minutes to a solution of 3-nitropyrazole (17.4 g.) in dry DMF (150 ml.) with external ice cooling to maintain the temperature at 20—30°C. The mixture was stirred for 45 minutes and to the almost clear solution was added 5-bromovaleronitrile (25 g.) over 30 minutes, at 25—30°C., and the mixture was stirred for 4 hours. Water (450 ml.) and EtOAc (450 ml.) was added and the upper layer was separated, dried (MgSO$_4$) and evaporated *in vacuo* to an oil which was a mixture of 5 - (3 - nitropyrazol - 1 - yl)valeronitrile and 5 - (5 - nitropyrazol - 1 - yl)valeronitrile. The oil was divided into two 15 g. portions which were fractionated on a silica column (3.5 cm. diameter×100 cm. long) eluted at 2 atmospheres by EtOAc/60—80°C. petroleum ether (3:7 v/v). The 1:5 isomer was eluted first followed by the 1:3 isomer. The 5 - (3 - nitropyrazol - 1 - yl)valeronitrile had m.p. 32—33°C.

To a solution of 5 - (3 - nitropyrazol - 1 - yl)valeronitrile (9.16 g.) in dry tetrahydrofuran (200 ml.) was added 5% w/w palladium on carbon (1.8 g.). The mixture was stirred at 20°C. under an atmosphere of hydrogen. 3.2 Litres of hydrogen were absorbed over 4 hours. The catalyst was filtered off and the filtrate was evaporated *in vacuo* to give 5 - (3 - aminopyrazol - 1 - yl)valeronitrile as an oil.

To a solution of 5 - (3 - aminopyrazol - 1 - yl)valeronitrile (7.0 g.) in acetonitrile (25 ml.) was added 2,2,2-trifluoroethylisothiocyanate (6.02 g.). After 15 minutes the solvent was evaporated *in vacuo* to give 5 - (3 - [3 - (2,2,2 - trifluoroethyl)thioureido]pyrazol - 1 - yl)valeronitrile as a white crystalline solid, m.p. 96—98°C.

The above thiourea (12.5 g.) was dissolved in 8M ammonia in EtOH (120 ml.). Mercuric oxide (12.8 g.) was added and the mixture was stirred at 20°C. for 30 minutes. The resulting mixture was filtered and the filtrate was evaporated *in vacuo* to give 5 - [3 - (2 - [2,2,2 - trifluoroethyl]guanidino)pyrazol - 1 - yl]valeronitrile as an oil. A sample of the oil was dissolved in acetone and 5 molecular equivalents of maleic acid was added. Ether was added to the resulting clear solution to produce the crystalline maleate, m.p. 123—125°C.

5 - [3 - (2 - [2,2,2 - Trifluoroethyl]guanidino)pyrazol - 1 - yl]valeronitrile (13 g.) was added over 10 minutes to concentrated sulphuric acid (65 ml.) with stirring. The resulting solution was kept at 20°C. for 18 hours then diluted with ice (300 ml.) and basified to pH 9 with 10.8 N sodium hydroxide. The mixture was extracted with EtOAc (3×200 ml.) and the extract was dried (MgSO₄) and evaporated *in vacuo* to an oil which crystallised. The crude material was recrystallised from EtOAc to give 5 - [3 - (2 - [2,2,2 - trifluoroethyl]guanidino)pyrazol - 1 - yl]valeramide, m.p. 130°C.

Example 1

ICI 162846 (either pure polymorph B or a mixture of polymorph A and polymorph B; 1.0 g.) was dissolved in a mixture of n-butanol (1.0 ml.) and toluene (4.0 ml.) at reflux temperature (112°C.). The solution was cooled to 20°C. at a rate of 10°C./hour. The crystalline product was isolated by filtration washed with toluene and dried at 50°C. It was pure polymorph A.

Example 2

ICI 162846 (a mixture of polymorph A and polymorph C; 1.0 g.) was dissolved in a hot mixture of n-butanol (1.0 ml.) and toluene (4.0 ml.). Solid calcium hydroxide (0.05 g.) was then added and the mixture was heated under reflux for 1 hour. The hot mixture was filtered and the filtrate allowed to cool to ambient temperature at a rate of 10°C./hour. The crystalline product was isolated by filtration, washed with toluene and dried at 50°C. It was pure polymorph A.

The starting material may be obtained as follows:—

ICI 162846 (60 g.) containing 2% w/w acetic acid was dissolved in methyl isobutyl ketone (300 ml.) at reflux (110°C.). The solution was allowed to cool to ambient temperature over 16 hours. At 80°C. the solution was seeded with an enriched sample of polymorph C. The crystalline product was isolated by filtration, washed with isobutyl methyl ketone and dried at 70°C. It was a mixture of polymorph A and polymorph C.

**Claims for the Contracting States: BE, CH, DE, FR, GB, IT, LI, LU, NL, SE**

1. Polymorph A of 5 - [3 - (2 - [2,2,2 - trifluoroethyl]guanidino)pyrazol - 1 - yl]valeramide, having a crystallographic purity of at least 90% and which is characterised by an attenuated total reflection (ATR) infra-red spectrum having bands at 1685 (ms), 1560 (w), 1540 (m), 1455 (w), 1060 (mw), 832 (m), 788 (w) and 365 (m) cm⁻¹.

2. Polymorph B of 5 - [3 - (2 - [2,2,2 - trifluoroethyl]guanidino)pyrazol - 1 - yl]valeramide, having a crystallographic purity of at least 90% and which is characterised by an attenuated total reflection (ATR) infra-red spectrum having bands at 1665 (ms), 1550 (s), 1445 (m), 1360 (w), 1215 (mw), 1070 (w), 1050 (mw), 990 (w), 935 (w), 822 (m), 380 (m) and 350 (mw) cm⁻¹.

3. A process for preparing polymorph A claimed in claim 1 from polymorph B or from a mixture of polymorph A and polymorph B characterised by:—

(a) heating the starting material in a diluent at or above a temperature of 107°C.;

(b) recrystallising the starting material from a solution in a solvent in which crystallisation begins above 65°C., and at a cooling rate slower than 1°C./minute; or

(c) dissolving the starting material in a boiling solution of ethyl acetate and seeding with a crystal of polymorph A at about 67°C.

4. A process for preparing polymorph B claimed in claim 2 from polymorph A or from a mixture of polymorph A and polymorph B characterised by recrystallising the starting material from water and seeding the solution with a crystal of polymorph B in the temperature range 27—37°C.

5. A process for preparing polymorph A claimed in claim 1 from a mixture containing polymorph C of 5 - [3 - (2 - [2,2,2 - trifluoroethyl]guanidino)pyrazol - 1 - yl]valeramide, characterised by dissolving the starting material in a solvent in which crystallisation begins above 65°C., treating this solution with an insoluble base, removing the insoluble base and allowing the solution to cool at a rate slower than 1°C./minute.

**Claims for the Contracting State: AT**

1. A process for preparing Polymorph A of 5 - [3 - (2 - [2,2,2 - trifluoroethyl]guanidino)pyrazol - 1 - yl]valeramide having a crystallographic purity of at least 90% and which is characterised by an attenuated total reflection (ATR) infra-red spectrum having bands at 1685 (ms), 1560 (w), 1540 (m), 1455 (w), 1060 (mw), 832 (m), 788 (w) and 365 (m) cm⁻¹ from polymorph B of 5 - [3 - (2 - [2,2,2 - trifluoroethyl]guanidino)pyrazol - 1 - yl]valeramide or from a mixture of polymorph A and polymorph B characterised by:—

(a) heating the starting material in a diluent at or above a temperature of 107°C.;

(b) recrystallising the starting material from a solution in a solvent in which crystallisation begins above 65°C., and at a cooling rate slower than 1°C./minute; or

(c) dissolving the starting material in a boiling solution of ethyl acetate and seeding with a crystal of polymorph A at about 67°C.

2. A process for preparing polymorph B having a crystallographic purity of at least 90% and which is characterised by an attenuated total reflection (ATR) infra-red spectrum having bands at 1665 (ms), 1550 (s), 1445 (m), 1360 (w), 1215 (mw), 1070 (w), 1050 (mw), 990 (w), 935 (w), 822 (m) 380 (m) and 350 (mw) cm$^{-1}$ from polymorph A or from a mixture of polymorph A and polymorph B characterised by recrystallising the starting material from water and seeding the solution with a crystal of polymorph B in the temperature range 27—37°C.

3. A process for preparing polymorph A having a crystallographic purity of at least 90% and which is characterised by an attenuated total reflection (ATR) infra-red spectrum having bands at 1685 (ms), 1560 (w), 1540 (m), 1455 (w), 1060 (mw), 832 (m), 788 (w) and 365 (m) cm$^{-1}$ from a mixture containing polymorph C of 5 - [3 - (2 - [2,2,2 - trifluoroethyl]guanidino)pyrazol - 1 - yl]valeramide, characterised by dissolving the starting material in a solvent in which crystallisation begins above 65°C., treating this solution with an insoluble base, removing the insoluble base and allowing the solution to cool at a rate slower than 1°C./minute.

**Patentansprüche fur die Vertragsstaaten: BE, CH, DE, FR, GB, IT, LI, LU, NL, SE**

1. Polymorph A von 5 - [3 - (2 - [2,2,2 - Trifluoroethyl]guanidino)pyrazol - 1 - yl]valeramid, welches eine kristallografische Reinheit von mindestens 90% besitzt und durch ein abgeschwächtes Total-reflexions-(ATR)-Infrarotspektrum mit Banden bei 1685 (mw), 1560 (w), 1540 (m), 1455 (w), 1060 (mw), 832 (m), 788 (w) und 365 (m) cm$^{-1}$ gekennzeichnet ist.

2. Polymorph B von 5 - [3 - (2 - [2,2,2 - Trifluoroethyl]guanidino)pyrazol - 1 - yl]valeramid, welches eine kristallografische Reinheit von mindestens 90% besitzt und durch ein abgeschwächtes Total-reflexions-(ATR)-Infrarotspektrum mit Banden bei 1665 (mw), 1550 (s), 1445 (m), 1360 (w), 1215 (mw), 1070 (w), 1050 (mw), 990 (w), 935 (w), 822 (m), 380 (m) und 350 (mw) cm$^{-1}$ gekennzeichnet ist.

3. Verfahren zur Herstellung von Polymorph A gemäß Anspruch 1 aus Polymorph B oder aus einem Gemisch von Polymorph A und Polymorph B, gekennzeichnet durch

(a) Erhitzen des Ausgangsmaterials in einem Verdünnungsmittel auf oder über eine Temperatur von 107°C;

(b) Umkristallisieren des Ausgangsmaterials aus einer Lösung in einem Lösungsmittel, in welchem die Kristallisation über 65°C beginnt, und mit einer Abkühlgeschwindigkeit von weniger als 1°C/min; oder

(c) Auflösen des Ausgangsmaterials in einer siedenden Lösung von Ethylacetat und Beimpfen mit einem Kristall von Polymorph A bei ungefähr 67°C.

4. Verfahren zur Herstellung von Polymorph B gemäß Anspruch 2 aus Polymorph A oder aus einem Gemisch von Polymorph A und Polymorph B, gekennzeichnet durch Umkristallisieren des Ausgangsmaterials aus Wasser und Beimpfen der Lösung mit einem Kristall von Polymorph B im Temperaturbereich von 27—37°C.

5. Verfahren zur Herstellung von Polymorph A gemäß Anspruch 1 aus einem Gemisch, das Polymorph C von 5 - [3 - (2 - [2,2,2 - Trifluoroethyl]guanidino)pyrazol - 1 - yl]valeramid enthält, gekennzeichnet durch Auflösen des Ausgangsmaterials in einem Lösungsmittel, in welchem die Kristallisation über 65°C beginnt, Behandeln dieser Lösung mit einer unlöslichen Base, Abtrennen der unlöslichen Base und Abkühlenlassen der Lösung mit einer Geschwindigkeit von weniger als 1°C/min.

**Patentansprüche fur den Vertragsstaat: AT**

1. Verfahren zur Herstellung von Polymorph A von 5 - [3 - (2 - [2,2,2 - Trifluoroethyl]guanidino)-pyrazol - 1 - yl]valeramid, welches eine kristallografische Reinheit von mindestens 90% besitzt und durch ein abgeschwächtes Totalreflexions-(ATR)-Infrarotspektrum mit Banden bei 1685 (mw), 1560 (w), 1540 (m), 1455 (w), 1060 (mw), 832 (m), 788 (w) und 365 (m) cm$^{-1}$ gekennzeichnet ist, aus Polymorph B von 5 - [3 - (2 - [2,2,2 - Trifluoroethyl]guanidino)pyrazol - 1 - yl]valeramid oder aus einem Gemisch von Polymorph A und Polymorph B, gekennzeichnet durch

(a) Erhitzen des Ausgangsmaterials in einem Verdünnungsmittel auf oder über eine Temperatur von 107°C;

(b) Umkristallisieren des Ausgangsmaterials aus einer Lösung in einem Lösungsmittel, in welchem die Kristallisation über 65°C beginnt, und mit einer Abkühlgeschwindigkeit von weniger als 1°C/min; oder

(c) Auflösen des Ausgangsmaterials in einer siedenden Lösung von Ethylacetat und Beimpfen mit einem Kristall von Polymorph A bei ungefähr 67°C.

2. Verfahren zur Herstellung von Polymorph B, welches eine kristallografische Reinheit von mindestens 90% besitzt und durch ein abgeschwächtes Totalreflexions-(ATR)-Infrarotspektrum mit Banden bei 1665 (ms), 1550 (s), 1445 (m), 1360 (w), 1215 (mw), 1070 (w), 1050 (mw), 990 (w), 935 (w), 822 (m), 380 (m) und 350 (mw) cm$^{-1}$ gekennzeichnet ist, aus Polymorph A oder aus einem Gemisch von Polymorph A und Polymorph B, gekennzeichnet durch Umkristallisieren des Ausgangsmaterials aus Wasser und Beimpfen der Lösung mit einem Kristall von Polymorph B im Temperaturbereich von 27—37°C.

3. Verfahren zur Herstellung von Polymorph A, welches eine kristallografische Reinheit von mindestens 90% besitzt und durch ein abgeschwächtes Totalreflexions-(ATR)-Infrarotspektrum mit Banden bei 1685 (ms), 1560 (w), 1540 (m), 1455 (w), 1060 (mw), 832 (m), 788 (w) und 365 (m) cm$^{-1}$ gekennzeichnet ist, aus einem Gemisch, welches Polymorph C von 5 - [3 - (2 - [2,2,2 - Trifluoroethyl]guanidino)pyrazol - 1 - yl]valeramid enthält, gekennzeichnet durch Auflösen des Ausgangsmaterials in einem Lösungsmittel, in welchem die Kristallisation über 65°C beginnt, Behandeln dieser Lösung mit einer unlöslichen Base, Abtrennen der unlöslichen Base und Abkühlenlassen der Lösung mit einer Geschwindigkeit von weniger als 1°C/min.

**Revendications pour les Etats Contractants: BE, CH, DE, FR, GB, IT, LI, LU, NL, SE**

1. Polymorphe A de 5 - [3 - (2 - [2,2,2 - trifluoroéthyl]guanidino)pyrazole - 1 - yl]valéramide ayant une pureté cristallographique d'au moins 90% et caractérisé par un spectre infrarouge avec réflexion totale atténuée (RTA) présentant des bandes à 1685 (ms), 1560 (w), 1540 (m), 1455 (w), 1060 (mw), 832 (m), 788 (w) et 365 (m) cm$^{-1}$.

2. Polymorphe B de 5 - [3 - (2 - [2,2,2 - trifluoroéthyl]guanidino)pyrazole - 1 - yl]valéramide ayant une pureté cristallographique d'au moins 90% et caractérisé par un spectre infrarouge avec réflexion totale atténuée (RTA) présentant des bandes à 1665 (ms), 1550 (s), 1445 (m), 1360 (w), 1215 (mw), 1070 (w), 1050 (mw), 990 (w), 935 (w), 822 (m), 380 (m) et 350 (mw) cm$^{-1}$.

3. Procédé de préparation du polymorphe A suivant la revendication 1 à partir du polymorphe B ou d'un mélange de polymorphe A et de polymorphe B, caractérisé par:

(a) le chauffage de la matière de départ dans un diluant à une température égale ou supérieure à 107°C;

(b) la recristallisation de la matière de départ dans sa solution dans un solvant dans lequel la cristallisation débute au-dessus de 65°C et à une vitesse de refroidissement inférieure à 1°C/minute; ou

(c) la dissolution de la matière de départ dans une solution bouillante d'acétate d'éthyle et l'ensemencement avec un cristal du polymorphe A à environ 67°C.

4. Procédé de préparation du polymorphe B suivant la revendication 2 à partir du polymorphe A ou à partir d'un mélange du polymorphe A et du polymorphe B, caractérisé par la recristallisation de la matière de départ dans l'eau et l'ensemencement de la solution avec un cristal de polymorphe B dans l'intervalle de température de 27 à 37°C.

5. Procédé de préparation du polymorphe A suivant la revendication 1 à partir d'un mélange contenant le polymorphe C du 5 - [3 - (2[2,2,2 - trifluoréthyl]guanidino)pyrazole - 1 - yl]valéramide, caractérisé par la dissolution de la matière de départ dans un solvant dans lequel la cristallisation débute au-dessus de 65°C, le traitement de cette solution avec une base insoluble, l'élimination de la base insoluble et le refroidissement de la solution à une vitesse inférieure à 1°C/minute.

**Revendications pour l'Etat Contractant: AT**

1. Procédé de préparation du polymorphe A du 5 - [3 - (2 - [2,2,2 - trifluoréthyl]guanidino)pyrazole - 1 - yl]valéramide ayant une pureté cristallographique d'au moins 90% et caractérisé par un spectre infrarouge avec réflexion totale atténuée (RTA) présentant des bandes à 1685 (ms), 1560 (w), 1540 (m), 1455 (w), 1060 (mw), 832 (m), 788 (w) et 365 (m) cm$^{-1}$ à partir du polymorphe B du 5 - [3 - (2 - [2,2,2 - trifluoréthyl]guanidino)pyrazole - 1 - yl]valéramide ou d'un mélange du polymorphe A et du polymorphe B, caractérisé par:

(a) le chauffage de la matière de départ dans un diluant à une température égale ou supérieure à 107°C.

(b) la recristallisation de la matière de départ dans une solution dans un solvant dans lequel la cristallisation débute au-dessus de 65°C et à une vitesse de refroidissement inférieure à 1°C/minute;

ou (c) la dissolution de la matière de départ dans une solution bouillante d'acétate d'éthyle et l'ensemencement à l'aide d'un cristal du polymorphe A à environ 67°C.

2. Procédé de préparation du polymorphe B ayant une pureté cristallographique d'au moins 90% et qui est caractérisé par un spectre infrarouge avec réflexion totale atténuée (RTA) présentant des bandes à 1665 (ms), 1550 (s), 1445 (m), 1360 (w), 1215 (mw), 1070 (w), 1050 (mw), 990 (w), 935 (w), 822 (m), 380 (m) et 350 (mw) cm$^{-1}$ à partir du polymorphe A ou d'un mélange de polymorphe A et de polymorphe B, caractérisé par la recristallisation de la matière de départ dans l'eau et l'ensemencement de la solution avec un cristal de polymorphe B dans l'intervalle de température de 27 à 37°C.

3. Procédé de préparation de polymorphe A ayant une pureté cristallographique d'au moins 90% et qui est caractérisé par un spectre infrarouge avec réflexion totale atténuée (RTA) présentant des bandes à 1685 (ms), 1560 (w), 1540 (m), 1455 (w), 1060 (mw), 832 (m), 788 (w) et 365 (m) cm$^{-1}$ à partir d'un mélange contenant le polymorphe C du 5 - [3 - (2 - [2,2,2 - trifluoréthyl]guanidino)pyrazole - 1 - yl]valéramide, caractérisé par la dissolution de la matière de départ dans un solvant dans lequel la cristallisation débute au-dessus de 65°C, le traitement de cette solution avec une base insoluble, la séparation de la base insoluble et le refroidissement de la solution à une vitesse inférieure à 1°C/minute.